# EUROPEAN PATENT APPLICATION

(11) **EP 4 450 969 A1**
(43) Date of publication of application: **23.10.2024**
(21) Application number: 23169211.2
(22) Date of filing: 21.04.2023
(51) Int. Cl.: G01N 33/50, C12M 1/12, D01F 6/00, G01N 1/22, C12N 5/00

(54) **METHOD OF ESTIMATING AEROSOL PARTICLES CYTOTOXICITY**

(71) Applicant: Kaunas University of Technology, 44249 Kaunas (LT); Centre for Innovative Medicine, 08406 Vilnius (LT)
(72) Inventor: Martuzevicius, Dainius, LT-44249 Kaunas (LT); Krugly, Edvinas, LT-44249 Kaunas (LT); Tichonovas, Martynas, LT-44249 Kaunas (LT); Dabasinskaite, Lauryna, LT-44249 Kaunas (LT); Masione, Goda, LT-44249 Kaunas (LT); Ciuzas, Darius, LT-44249 Kaunas (LT); Prasauskas, Tadas, LT-44249 Kaunas (LT); Kauneliene, Violeta, LT-44249 Kaunas (LT); Bagdonas, Edvardas, LT-08406 Vilnius (LT); Aldonyte, Ruta, LT-08406 Vilnius (LT); Raudoniute, Jovile, LT-08406 Vilnius (LT)
(74) Representative: Klimaitiene, Otilija

(57) **Abstract**

The invention discloses a method and an application of a fibrous matrix as an aerosol sampling filter (14) in toxicological analysis of collected particles, i.e., aerosol particle cytotoxicity studies. It is based on a single platform - fibrous matrix (15, 23) for aerosol particle sampling/collection, and subsequent analysis of collected particles for their cytotoxic properties, by seeding cells (33) directly on collected particles (21). Such method allows for a simpler, faster and less expensive procedure, and is capable in a more accurate representation of aerosol particle cytotoxicity associated with both soluble and insoluble particles. The platform consists of polymer fibre composite matrix 14) possessing nanofibrous top layer (13) and allowing collection of nanoparticles on top of this layers. The membrane also supports cell attachment and proliferation as a fibrous 2D scaffold for cell (33) cultivation. Aerosol particle (21) cytotoxicity is estimated based on the monitoring of the cell (33) viability/stress and measuring other cellular health biomarkers. Such method may be applied for the determination of aerosol particle cytotoxicity in indoor occupational, residential, hospitality environments, and outdoor air.

## Description

### FIELD OF INVENTION

The present invention discloses a method employing a novel "cells-on-particles" concept to determine the toxicity (namely, cytotoxicity) of ambient aerosol particles. The aerosol particle cytotoxicity provides an important information in ambient air pollution, public health, and exposure sciences.

### BACKGROUND ART

Fine particles suspended in ambient air (aerosol particles) are known to cause adverse affect to human health. Depending on their size, they penetrate human airways and get settled in the upper respiratory tract (referred to as inhalable aerosol fraction), the middle respiratory tract (thoracic fraction), and the lower respiratory tract (respirable fraction). The latter fraction of aerosol particles is of special importance, since due to a small size it reaches lungs, enters the alveoli, and subsequently, bloodstream. Aerosol particles increase the incidence of various diseases such as asthma, cardiovascular, cancer, and others. Therefore, understanding the causes of the adverse health effects is of paramount importance in current human exposure studies [1].

*In vitro* assays serve as a cost-effective, ethically favourable, and informative analysis for the observation and estimation of toxicity and its effects on cells [2]. Typically, *in vitro* assays rely on two main approaches: indirect (submerged exposure) and direct (air-liquid interface) [3].

Direct methods involve the deposition of aerosol particles directly onto cultured cells via a specially prepared membrane (i.e., air-liquid interface), allowing to physiologically mimic the inhalation process over a defined period. This way the method is advantageous over the indirect method since the dose of particles can be determined more precisely [4][5][3]. However, the direct air-liquid interface method mostly aims to quantify inhalation toxicology endpoints, as opposed to general cytotoxicity. The method is based on directing aerosol stream onto monolayer of specially cultivated cells, placed in a specific exposure chamber. Such method provides the toxicological response to the real-time unaltered aerosol particles.

The international patent application WO2011094692A2 *"Systems and methods for collecting and depositing particulate matter onto tissue samples"* discloses direct aerosol *in vitro* exposure systems and methods based on the electrostatic charge of particles. These systems can be used for collecting and depositing particulate matter onto tissue without pre-concentration, and without intermediate collection steps such as use in water or on a filter. The system can include an inlet configured to receive air containing particulate matter, a receptacle configured to hold one or more tissue samples, a porous membrane providing support for an air-liquid interface of the tissue sample, and an electrostatic precipitation area. Particulate matter contained in the air received at the inlet can be electrically charged in the electrostatic precipitation area and flowed over the tissue sample, where it can be collected and measured.

The US patent application US2018171280A1 *"Cell culture exposure system* (cces)" discloses a direct aerosol in vitro exposure system - an air-liquid interface (ALI) exposure of cultured cells. A method for evaluating effect of a polluting air stream comprises the steps of
1) exposing cells to a membrane until adhesion of the cells has occurred;
2) feeding cells periodically until there is a confluent monolayer of cells on said membranes;
3) aspirating off non-adherent cells, applying fresh media;
4) exposing the cells to over-head stream containing pollutants;
5) removing the cells from the membranes that have been exposed to the over-head stream,
6) measuring the cells' response to toxins in the over-head stream.

The indirect method is based on the following sequence of operations:
1) collecting aerosol particles onto sampling substrates or filters;
2) recovering aerosol particles from a filter using extraction to a liquid (usually phosphate buffer solution, methanol or Gamble's solution);
3) Filtering the extract from remaining insoluble particles;
4) placing extract into culture medium containing cells;
5) Recording cell response.

This (indirect) method is comparatively simpler than the direct method, since it does not require specific equipment for exposing cells. A major drawback of the indirect method that it only indicates the toxicity of soluble particles that dissolve in the liquid media during the extraction process. This leads in an underestimation of toxicity [4][5].

The Chinese patent application CN102346147A *"Method for detecting difference of cell toxicity between atmospheric nano particles and industrial nano particles"* relates to a an indirect method for detecting the difference of cell toxicity between atmospheric nano-particles and industrial nano-particles. The method comprises the following steps of:
1) using a culture medium to prepare a cell suspension, inoculating the cell suspension in a cell culture dish and culturing the culture dish in an incubator for 24 hours;
2) mixing a prepared particulate matter solution and the culture medium and preparing into a contaminated solution; using a D-hank's balanced salt solution to clean cells; carrying out contamination on the cells, and culturing the contaminated cells in the incubator for 4 hours;
3) adding a mixed solution of a DCFH-DA fluorescent probe and the culture medium, sealing and wrapping with tin foil paper, and culturing the mixed solution in the incubator for 0.5 hour;
4) observing the fluorescence intensity and the fluorescence distribution under an inverted fluorescence microscope, and photographing; and
5) processing images by fluorescence analysis software to obtain initial data, analysing and comparing.

According to the above method, the cell section preparation is fast and simple, and the raw material is easy to obtain; by the cell detection method, the result is accurate, the influence factor is fewer, and multiple nano-particles can be detected simultaneously; and laboratory reagents are safe and reliable and have no strong toxicity. The method does not include the procedure for the collection of aerosol particles. It may be assumed that this procedure involves collection on certain filter and subsequently extracting particles into a solution, which makes it a classical indirect method.

The above presented information indicates that the determination of aerosol particle cytotoxicity is a complex multistage procedure, both by indirect and direct methods, thus the simplification of this procedure is highly appreciated [1].

Current direct methods involve deficiencies including:
1) Cytotoxicity determination based on inhalation toxicology endpoint only;
2) Preparation of cell monolayer is complicated and time consuming;
3) Costly method due to the requirement of specific equipment, i.e., exposure chambers.

Current indirect methods involve deficiencies including:
1) a long aerosol particle collection time. Usually, high-volume sampling is necessary, i.e., at least 900 m³ of air to collect sufficient amount of fine particulate matter to result in a toxicological response;
2) transfer of particles from a filter substrate by solvent extraction, dissolve and filter residual particles. Such sequence requires the utilization of time and equipment. Moreover, due to the partial solubility of particulate matter in a selected solvent, only partial information of particle cytotoxicity is obtained [6].

### SUMMARY OF INVENTION

**Technical problem.** The background of the technical problem is that determination of aerosol particle cytotoxicity involves an overly complicated procedure, as presented in the state of art, comprising of long duration of particle sampling, multiple stages of processing, or a complicated exposure equipment. Another important problem is associated with the fact that methods of determining aerosol particle cytotoxicity that rely on the extraction of particle from the sampling substrate may misinterpret the true toxicity potential, since the extracted suspension carries only a partial information on the particle's cytotoxicity.

**Solution.** This invention provides a more rapid and simpler integrated method for an aerosol particle cytotoxicity testing directly from filter media where particles are collected. Moreover, it provides a superior estimate of particle's cytotoxicity, associated with their physical shape and chemical composition, which is unaltered by the solvent extraction stage.

The method is based on a single integrated polymer fibrous platform for particle sampling/collection and a subsequent cell cultivation. Such approach results in a simpler procedure, a more representative resulting data and a faster observation rate compared to *in vitro* models that are based on particle extracts, or air-liquid interface. The invention extends the use of aerosol sampling filters by specifically designing these to be used in toxicological analysis of collected particles, i.e., aerosol particle cytotoxicity studies.

The fibrous platform consists of three fibrous layers: Layer 1 - the bottom layer composed of microfibres, providing structural support to the upper layers; Layer 2 - the middle layers of nanofibers, providing good adhesion between the bottom and top layers; and Layer 3 - the top layer composed of nanofibers, providing an efficient 2D surface for collecting aerosol particles as well as cultivating cells to observe the cytotoxic response. "2D surface" in this context means that cells proliferate on the top of the matrix (on the top-layer, Layer 3) and do not migrate into Layer 2 and Layer 1. The three-layer-structure forms a fibrous matrix that features high mechanical stability during aerosol sampling and subsequent handling and *in vitro* cell cultivation. Moreover, it ensures good circulation of cell culture media within the fibrous matrix, as large pores of the microfibre layer (Layer 1) provide low resistance for culture media to approach upper layers, thus providing nutrition to cells from the direction of attachment, not only from the top.

The technique and process presented provides an intermediate approach between existing indirect and direct methods for the cytotoxicity detection of aerosol particles. Similarly to the direct method, aerosol particles are collected on a filter, but the extraction, purification, and suspension steps of the collected particles are avoided. The similar feature to the direct method is in the fact that the fibrous matrix with particles is immersed in a culture medium and cells are placed on top of the matrix. The cells thus have a stable 2D-support-scaffold in which the collected particles remain on the surface of the fibrous matrix, thus ensuring more effective contact with the cells, compared to the usual direct method.

**Advantageous effects.** The invention presents an improved and simpler method for the determination of particle cytotoxicity. The application of the method simplifies the cytotoxicity testing method as it provides a single platform (fibrous matrix) for aerosol particle collection and cell cultivation, by placing cells directly on collected particles. This is possible to achieve by using a unique design of an aerosol sampling filter (composite three-layer fibrous matrix), that features a top nanofibrous layer, which serves as an efficient filter for aerosol particle collection, but also as a 2D-fibrous cell cultivation scaffold.

Said method also results in a more representative cytotoxicity assay since cells under cytotoxicity test are exposed closely and directly to un-dissolved collected aerosol particles as well as to the dissolved particles in the water-based cultivation media thus carrying more accurate information on cytotoxicity, as opposed to the traditional indirect testing, where cells are exposed to a solution of soluble fractions of aerosol particles.

Furthermore, said method speeds-up the process of particle cytotoxicity testing thus making it efficient in terms of time and material usage. The analysis time is reduced by skipping steps of particle extraction from the filter, dissolution, filtering, and suspension that are included in the indirect procedure, as well as preparation of monoculture and complex set-up that are featured in the direct procedure. The method saves materials that are applied routinely for aerosol particle cytotoxicity testing (such as extraction solvents, filtering media, syringe consumables), thus reducing not only economic costs, but also the negative impact on the environment associated with the usage of chemicals or laboratory consumables.

### DESCRIPTION OF DRAWINGS

The drawings are provided as a reference to possible embodiments and are not intended and should not limit the scope of the invention.

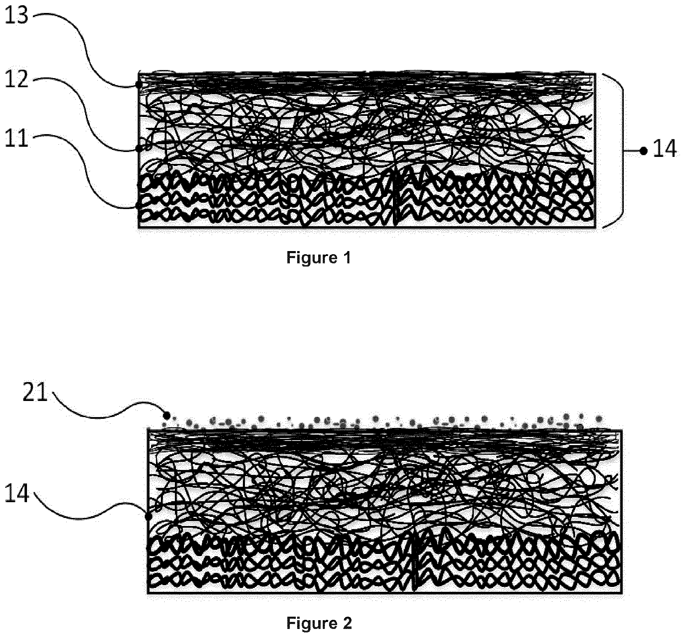

- **Figure 1.**: shows a conceptual depiction of the composition of three-layer fibrous matrix 11- Layer 1 serving as a structural microfibrous support for the layers 2 and 3; 12 - Layer 2 serving as a mesofibrous binding layer between the Layer 1 and the nanofibrous Layer 3; 13 - Layer 3 serving as the working nanofibrous surface for the collection of aerosol particles; 14 - integrated three-layer fibrous matrix.
- **Figure 2.**: shows a an application of three-layer fibrous matrix as an aerosol sampling filter: 14 - integrated three-layer fibrous matrix, serving as an aerosol sampling filter; 21 - aerosol particles collected on the surface of the sampling filter.

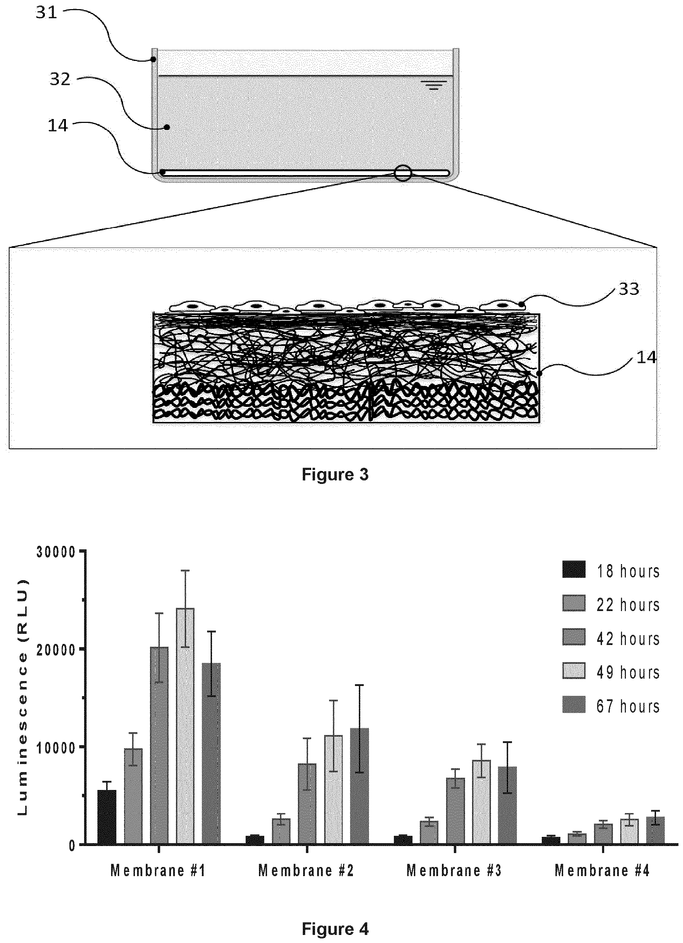

- **Figure 3.**: shows a an application of three-layer fibrous matrix as a 2D cell cultivation substrate: 14 - integrated three-layer fibrous matrix, serving as a fibrous 2D cell cultivation substrate; 31 - a single well in a microwell plate; 32 - cell culture media; 33 - cells placed on the top layer of the cell cultivation substrate.
- **Figure 4.**: shows the assessment of proliferation and viability of Beas-2B cells on fibrous matrix measured by applying the RealTime-Glo MT Cell Viability Assay (Promega corp.) and using a continuous-read format protocol. Membrane #1 represents a fibrous matrix fabricated of poly[ε]caprolactone Layer 1, cellulose acetate Layer 2, and cellulose acetate Layer 3. Membrane #2 represents a fibrous matrix fabricated of poly[ε]caprolactone Layer 1, poly[ε]caprolactone Layer 2, and poly[ε]caprolactone Layer 3. Membrane #3 represents a fibrous matrix fabricated of poly[ε]caprolactone Layer 1, poly[ε]caprolactone Layer 2, and polyacrylonitrile Layer 3. Membrane #4 represents a fibrous matrix fabricated of poly[ε]caprolactone Layer 1, poly[ε]caprolactone Layer 2, and polyamide Layer 3.

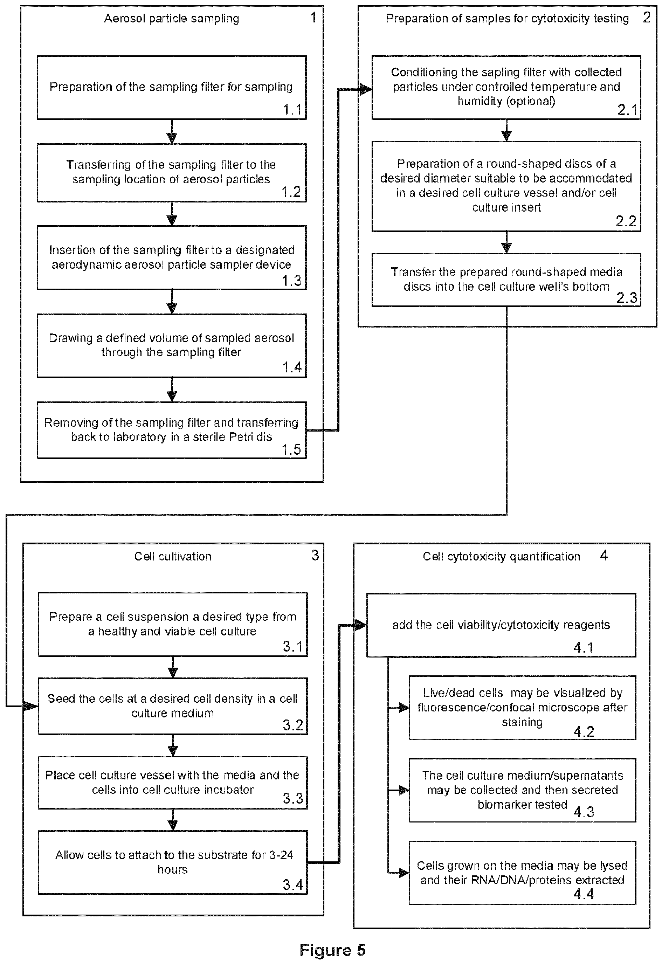

- **Figure 5.**: shows the flowchart of the method application.

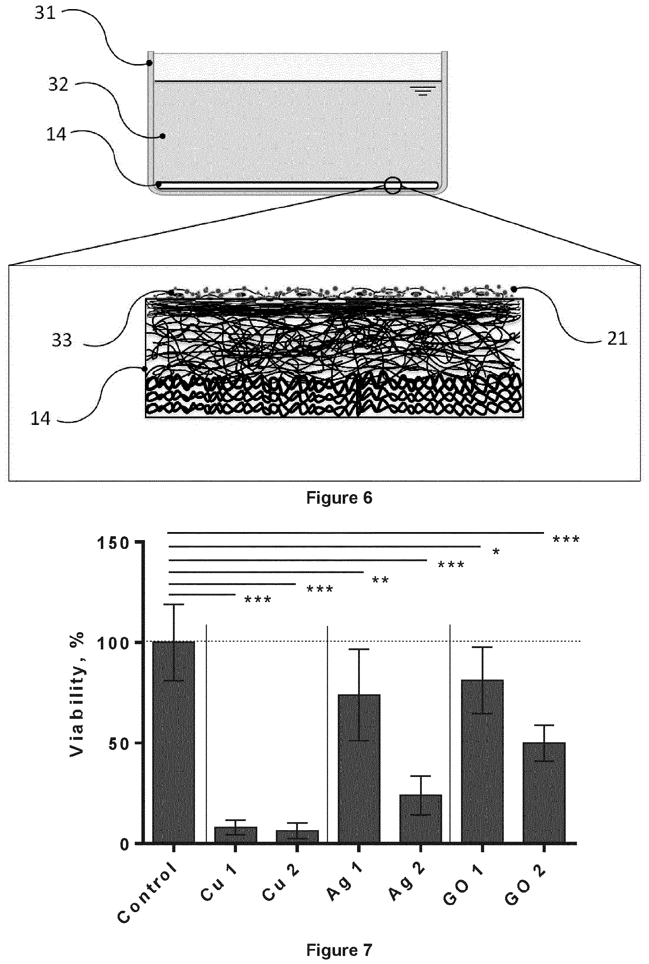

- **Figure 6.**: shows a an application of three-layer fibrous matrix as an integrated platform for the determination of aerosol particle cytotoxicity: 14-integrated three-layer fibrous matrix, serving as an aerosol particle collection filter and subsequently, as a fibrous 2D cell cultivation substrate; 51 - cell culture media; 52 - particles collected from air; 53-cells placed on the fibrous matrix and collected particles.

- **Figure 7.**: shows cytotoxicity response (as cell viability) versus the amount of aerosolized and deposited copper (Cu), silver (Ag), and graphene oxide (GO) nano-particles, as determined using the proposed method. Each type of nanoparticles was tested with low and high dose. Cu1 represents deposited dose of nanoparticles of 3.87 µg/cm² (lower dose), Cu2 -13.96 µg/cm² (higher dose), and respectively, Ag1 - 8.42 µg/cm², Ag2 - 19.46 µg/cm², GO1 - 0.43 µg/cm², GO2 - 2.72 µg/cm². Control was assessed with a blank fibrous matrix.

### DETAILED DESCRIPTION OF INVENTION

The object of the invention is a method for the determination of aerosol particle cytotoxicity *in vitro* based on the direct placement of cells on aerosol particles collected on a specially prepared fibrous matrix - an aerosol sampling filter, which also serves as a fibrous 2D cell cultivation platform.

**The usage of the fibrous matrix as a an aerosol particle sampling filter.** This filter is constructed to possess several key features:
1) Must collect aerosol particles at a high efficiency (>99.5% for respirable aerosol particles).
2) Must possess sufficient mechanical rigidity to maintain its shape during handling, particle sampling, and *in vitro* cell seeding experiments.
3) Must be biocompatible and not cytotoxic;
4) Must support cell attachment and proliferation, during at least 72 hour period;

The design and fabrication methods of these sampling filters has been disclosed in the Lithuanian patent application LT2021 574, national filing date 2021-12-10, and the European patent application EP22170405.9, EPO filing date 28 April 2022).

The aerosol sampling filter is constructed as a composite filter disc comprising at least three non-woven fibrous layers. The conceptual depiction of the cross-section of such composite filter is presented in Figure 1.

The three-layer composition ensures key operational parameters of the sampling filter, namely, good mechanical stability, high-efficiency of particle collection, and optimum pressure drop across the filter layer. Due to a small fibre and pore size as well as a high packing density of nanofibre network of the top layer (as shown in Figure 2), the entire composite works primarily as a surface filter where particles are collected above the fibres (as opposed to volume filters, where particles penetrate deeply into the filtering matrix). The prevailing particle capture mechanisms include impaction, interception, and diffusion.

This filter is sized accordingly to the sampling device that the filter is hosted in. The three-layered structure of such composite filter is unique and superior to just nanofibre membrane in terms of higher mechanical stability and lower pressure drop.

Layer 1 (the bottom layer) comprises a microfibre network and serves as a mechanically robust support for the upper nanofibre layers. This layer must possess the following technical parameters: fibre diameter in a range between 20 and 50 µm, pore size in the range between 30 and 200 µm; fibre orientation is random or semirandom (intermixed rectangularly oriented and randomly oriented fibres).

Layer 2 (the middle layer) comprises larger nanofibre network and ensures a biding between the to and the bottom layer, as well as the adequate mechanical support for the fragile upper layer. This layer must possess the following technical parameters: fibre diameter in a range between 0.7 and 5 µm, pore size in the range between 1 and 10 µm; fibre orientation is random.

Layer 3 (the top layer) acts as the working layer which provides a nanofibre network for the collection of aerosol particles on the surface. This layer must possess the following technical parameters: fibre diameter in a range between 0.08 and 0.5 µm, pore size in the range between 0.1 and 3 µm; fibre orientation is random.

The surface roughness (measured as average roughness as the deviation of a surface from a mean height) of such composite layered filter ranges between 0.1 and 0.8 µm. The thickness of such composite layered filter varies between 50 and 500 micrometres, depending on the materials that the layer is comprised of.

The following polymers may be used to fabricate layers of said filter, but not limited to:
- Poly(methacrylic acid methyl ester) (PMMA),
- Poly(hexano-6-lactam) (PA6),
- Poly(1-acrylonitrile), (PAN),
- Cellulose acetate (CA),
- Poly(hexano-6-lactone) (PCL),
- Poly(lactic acid) (PLA).

The fabrication technique of Layer 1 is based on the method for the formation of polymer fibre matrixes by the combined fused deposition model and electrospinning. The fabrication technique of Layer 2 and Layer 3 is based on a liquid-solution electrospinning.

**The usage of the fibrous matrix as a 2D cell cultivation platform.** The present method employs these fibrous matrixes also as scaffold or membrane for *in vitro* cell cultivation substrates. In terms of *in vitro* cell cultivation, the three-layer composition ensures key operational parameters of the fibrous 2D cell cultivation surface, namely, no intrinsic cytotoxicity, low surface roughness, small pores, and good mechanical stability in terms of handling and as submersed in cell cultivation media.

The application of the substrate as a fibrous 2D cell cultivation platform has been proven using human non-tumorigenic lung epithelial cell line (BEAS-2B,

Figures 3 and 4). The cells were placed on a clean substrate following procedure presented below, and cultivated for a defined period of time, followed by the RealTime-Glo MT Cell Viability Assay (Promega) measurements. This method estimates cell viability by measuring the amount of luminescence emitted from cells that have been exposed to a cell-permeant form of Glo reagent. The luminescence signal is a direct measure of the amount of Adenosine triphosphate (ATP) produced by the cells, which is proportional to the number of viable cells. Therefore, higher luminescence signals indicate higher cell viability and lower luminescence signals indicate lower cell viability.

The luminescence signal in Beas-2B cells increased over time, showing proliferation and viability on all the types of the membranes tested. Membrane #4 - with the upper layer fabricated of polyamide 6 resulted in the lowest luminescence signal, meaning the lowest proliferation rate among tested matrixes. Membrane #1 with the top layer fabricated of cellulose acetate resulted in the highest signal among all membranes, indicating the highest proliferation/metabolic activity.

**The usage of the fibrous matrix as a platform for the cytotoxicity testing of aerosol particles.** This method is advantageous to determine aerosol particle cytotoxicity in that it provides an optimized method based on a single integrated platform for aerosol particle collection and cell cultivation, thus minimizing the steps required for sample extraction, dissolution and transfer to cells (in case of a indirect method) or avoiding a complicated sampling setup (in case of a direct method).

The method may serve as a method to determine cytotoxicity of aerosol sampled from the variety of environments including but not limited to indoor occupational air, indoor residential air, indoor hospitality air, indoor public air, atmospheric urban air, and atmospheric rural air.

The proposed method allows determining the cytotoxicity of a broad variety of aerosol particles including but not limited to engineered nanoparticles, combustion aerosol particles, secondary organic aerosol particles, or tobacco product aerosol particles.

**Process and steps of the method.** The procedure of utilizing composite fibrous aerosol particle sampling filter for the collection and *in vitro* toxicity testing of aerosol particles contains of the following steps (Figure 5):
**1.** Aerosol particle sampling:
   1.1. Preparation of the fibrous matrix (sampling filter) for sampling, namely conditioning under controlled temperature (20-22 °C) and humidity (40-50%), followed by weighing in a microbalance;
   1.2. Transferring of the sampling filter to the sampling location of aerosol particles, by placing it in sterile Petri dish;
   1.3. Insertion of the sampling filter to a designated aerodynamic aerosol particle sampler device, including but not limited to a cassette, an impactor, or a cyclone;
   1.4. Drawing a defined volume of sampled aerosol through the sampling filter using a vacuum pump at a defined flow rate specific to the sampling device. The volume of air drawn must be determined according to the concentration of aerosol particle in ambient/occupational environment or emission process. The goal is to obtain the deposit of particles within the interval of 20 to 500 ug, depending on the expected particle cytotoxicity. The existing aerosol particle concentration may be estimated by carrying out an initial aerosol sampling or measuring real-time aerosol particle concertation using a direct reading instrument;
   1.5. Removing of the sampling filter and transferring back to laboratory in a sterile Petri dish, preferably under freezing conditions (-18 °C) to prevent evaporation of samples semi-volatile species.
**2.** Preparation of collected samples of fibrous matrix containing particles for cytotoxicity testing:
   2.1. Conditioning the fibrous matrix with collected particles under controlled temperature and humidity, followed by weighing. This stage is optional, and best implemented in case a parallel sampling is performed on another filtering substrate that allows determining the mass of particles collected on sampling filter. It must be noted that during the conditioning some volatile chemical species may evaporate from the collected particles thus altering their cytotoxicity;
   2.2. Preparation of a circle-shaped discs of a desired diameter suitable to be accommodated in a desired cell culture well/vessel and/or cell culture insert (including but not limited to 96-, 48-, 24-, 12- well plate format etc.). Procedure is performed under sterile conditions in the laminar flow hood, with a sterile mechanical hole puncher. Discs are stored until cytotoxicity tests under freezing conditions (-18 °C) to prevent the evaporation of volatile organic species present in particles.
   2.3. Transfer the prepared round-shaped matrix discs into the cell culture well's bottom of the sterile cell culture plate of a defined and suitable diameter. The plate needs to be a "cell low binding" type (i.e. chemically treated or covered with a specific polymers, including but not limited to polyHEMA or similar). Alternatively, the circle-shaped fibrous matrix discs might be placed into cell culture inserts and then into the culture plates. Procedures are performed in sterile conditions (cell culture flow air laminar).
**3.** Cell cultivation
   3.1. Prepare a cell suspension from a healthy and viable cell culture, following a standardized sequence, such as thawing the cells if they are frozen; centrifuging the cells to pellet them; discarding the supernatant, and resuspending the cells in a suitable growth medium; counting the cells using a hemacytometer or automated cell counter, and adjusting the cell density to the desired concentration; transferring the cell suspension to a sterile culture vessel; incubating cells at the appropriate temperature, humidity, and CO₂ level; harvesting for seeding, when the cells reach the desired density or confluency.
   3.2. Seed the cells of a desired type at a desired cell density in a cell culture medium supplemented with but not limited to fetal bovine serum (FBS)/fetal calf serum (FCS), antibiotics/antimycotics directly onto the matrix.
   3.3. Place cell culture vessel with the fibrous matrix and the cells into cell culture incubator with a specific temperature, humidity and air (including 37°C, 21 % O₂, 5 % CO₂, but not limited to).
   3.4. Allow cells to attach to the substrate for 3-24 hours, but not limited to. If needed, cell culture medium/reagents might be added at any time depending on the testing procedures.
**4.** Cell cytotoxicity quantification
   4.1. Depending on the objectives of the experiment, add the cell viability/cytotoxicity reagents, cell dyes into the wells at a desired time and perform the readings of the signals directly from the culture wells by spectrometer (absorbance, fluorescence, luminescence) or collect/transfer the medium/supernatants from the cultured cells on the matrix for viability/cytotoxicity etc. tests.
   4.2. Live/dead cells on the matrix may be visualized by fluorescence/confocal microscope after staining.
   4.3. The cell culture medium/supernatants may be collected and then secreted biomarker tested.
   4.4. Cells grown on the matrix may be lysed and their RNA/DNA/proteins extracted for a desired biomarker analysis, including gene/protein expression, DNA modifications, but not limited to.

**Experimental results.** The effect of aerosolized nanoparticles collected on the fibrous matrix was evaluated with BEAS-2B cells (Figures 6 and 7). Three types of nanoparticles were tested, including elemental copper (denoted as Cu, particle size 60-80 nm), elemental silver (Ag, <100nm), and graphene oxide (GO, average no. of layers 15-20). The effects of nanoparticles to cells were recorded using a continuous-read format, i.e. the addition of assay reagents at the beginning of cell exposure and then measuring the luminescence signal at a desired time points directly from the wells.

The luminescence signal in BEAS-2B cells increased over time, indicating proliferation of cells over time. The exposed cells to nanoparticles showed a decreased signal comparing to a relative control cells witch significantly differed from the controls 40 and 48 hours after exposure/cell seeding. The decreased luminescence shows the reduction in the number of viable cells and/or reduced proliferation. This indicates that the method is capable in quantitatively detecting changes in cell activity based on the amount of deposited aerosol particles.

**Embodiments.** The presented method implemented following the procedure described in the Method application, which provides an embodiment scenario in case of ambient or indoor aerosol sampling. The choice of an aerodynamic aerosol sampler described in the method stage 3 should be restricted to low flowrate devices (1-30 lpm), as higher flowrates may cause high pressure drop and mechanical damage of the membrane. The composite membrane should tightly the fit the sampler filter holder, which is usually 25 mm, 37 mm or 47 mm in diameter. Potential sampler may include filter based samples, such as Filter Cassette, Clear Styrene, 37 mm (SKC Inc, USA), Stainless steel or Teflon coated cyclones (URG corp., USA), Personal Environmental Monitor (SKC Inc., USA), Low-volume air sampler (Met One Instruments Inc, USA) or impactor based samplers, such as MOUDI Rotating Impactors (TSI INC, USA), Electrostatic low pressure impactor (ELPI, Dekati Ltd, Finland), and Sioutas Personal Cascade Impactor (SKC Inc, USA), but not limited to these types of samplers only.

The proposed method allows a broad selection of cell lines for the estimating of particle cytotoxicity, described in Stage 9, going beyond human lung-derived cell lines. This selection may include any type of adherent cells derived from tissues of organs, such as, but not limited to:
- BEAS-2B cells (human bronchial epithelial cells);
- A549 cells (human lung carcinoma cells);
- Calu-3 cells (human lung adenocarcinoma cells);
- 16HBE14o- cells (human bronchial epithelial cells);
- NCI-H292 cells (human pulmonary mucoepidermoid carcinoma cells);
- HepG2 cells (human liver carcinoma cells);
- HaCaT cells (human immortalized keratinocytes);
- HUVEC cells (human umbilical vein endothelial cells);
- RAW 264.7 cells (murine macrophage cells);
- SH-SY5Y cells (human neuroblastoma cells);
- NRK-52E cells (rat kidney epithelial cells).

Live/dead cells staining for microscopy as described in stage 13 may be achieved with Calcein AM or Live/Dead staining kit. Cell viability/cytotoxicity tests may be applied including MTT, XTT, WST-8, Alamar Blue, LDH release, RealTime-Glo Cell Viability Assay, CellTiter-Glo Assay, CytoTox-Glo Assay, CellTox Green Assay, but not limited to.

Secreted biomarkers as described in stage 14 may be tested by means of ELISA, Luminex assays, but not limited to.

Extracted RND/DNA/proteins from the exposed cells as described in stage 15 may be tested by means of PCR/RTq-PCR, RNA/DNA-sequencing, Western Blot, Luminex, but not limited to.

**Applications of the method.** The presented method represents a relatively simple and fast method to be used in environmental laboratories, companies, or health institutions where the toxicity of the ambient air constituents should be estimated. Applications thereof are intended for estimating the toxicity of the ambient air constituents in environmental or occupational pollution research and investigations.

### NON-PATENT LITERATURE

[1] S. Schmidt, R. Altenburger, and D. Kühnel, "From the air to the water phase: implication for toxicity testing of combustion-derived particles," Biomass Convers Biorefin, vol. 9, no. 1, pp. 213-225, Mar. 2019, doi: 10.1007/s13399-017-0295-1.
[2] A. Sengupta et al., "A New Immortalized Human Alveolar Epithelial Cell Model to Study Lung Injury and Toxicity on a Breathing Lung-On-Chip System," Frontiers in Toxicology, vol. 4, p. 68, Jun. 2022, doi: 10.3389/ftox.2022.840606.
[3] S. Mülhopt et al., "Toxicity testing of combustion aerosols at the air-liquid interface with a self-contained and easy-to-use exposure system," J Aerosol Sci, vol. 96, pp. 38-55, Jun. 2016, doi: 10.1016/j.jaerosci.2016.02.005.
[4] W. W. Polk, M. Sharma, C. M. Sayes, J. A. Hotchkiss, and A. J. Clippinger, "Aerosol generation and characterization of multi-walled carbon nanotubes exposed to cells cultured at the air-liquid interface," Part Fibre Toxicol, vol. 13, no. 1, p. 20, Dec. 2015, doi: 10.1186/s12989-016-0131-y.
[5] J. S. Kim, T. M. Peters, P. T. O'Shaughnessy, A. Adamcakova-Dodd, and P. S. Thorne, "Validation of an in vitro exposure system for toxicity assessment of air-delivered nanomaterials," Toxicology in Vitro, vol. 27, no. 1, pp. 164-173, Feb. 2013, doi: 10.1016/j.tiv.2012.08.030.
[6] L. Liu, Q. Zhou, X. Yang, G. Li, J. Zhang, X. Zhou, and W. Jiang, "Cytotoxicity of the soluble and insoluble fractions of atmospheric fine particulate matter," Journal of Environmental Sciences, vol. 91, pp. 105-116, May 2020., doi.org/10.1016/j.jes.2020.01.012

## Claims

1. A method of cytotoxicity estimation, comprising at least steps of
1) sampling of aerosol particles (21) by a fibrous matrix (14);
2) seeding and cultivating cells (33) onto said fibrous matrix (14) containing aerosol particles (21);
3) quantifying the cultivated-cells cytotoxicity by cell viability/cytotoxicity reagents and analysis means,
w her e i n the fibrous matrix (14) used across the method steps 1) to 3) as a single composite fibrous matrix platform (14) comprising at least a nanofibrous top-layer (13), which allows collection of aerosol particles (21) as the particle sampling filter as well as seeding, cultivating and quantifying cells (33) on top of said nanofibrous top-layer (13).

2. The method according to claim 1, **wherein** the composite fibrous matrix (14) platform used in the method as the aerosol particle (21) sampling filter comprises:
• Layer 1 which is the bottom layer (11) comprising of microfibres,and providing structural support to the upper layers of the matrix (14);
• Layer 2 which is the middle layer or layers (12) of microfibres, providing adhesion between the bottom (11) and top (13) layers of the matrix (14); and
• Layer 3 which is the top-layer (13) composed of nanofibres, providing an efficient 2D surface for collecting aerosol particles as well as cultivating cells to observe the cytotoxic response thereof.

3. The method according to claim 2, wherein
• Layer 1 microfibres have fibre diameter in a range between 20 and 50 µm, pore size in the range between 30 and 200 µm; fibre orientation is random;
• Layer 2 microfibres have fibre diameter in a range between 0.7 and 5 µm, pore size in the range between 1 and 10 µm; fibre orientation is random; and
• Layer 3 nanofibres have fibre diameter in a range between 0.08 and 0.5 µm, pore size in the range between 0.1 and 3 µm; fibre orientation is random.

4. The method according to any claim of 1 to 3, **wherein** the composite fibrous matrix (14) fibrous structures of layers (11, 12, 13) are any of the materials:
- Poly(methacrylic acid methyl ester) (PMMA),
- Poly(hexano-6-lactam) (PA6),
- Poly(1-acrylonitrile), (PAN),
- Cellulose acetate (CA),
- Poly(hexano-6-lactone) (PCL),
- Poly(lactic acid) (PLA).

5. The method according to any claim of 1 to 4, **wherein** the surface roughness of such composite layered filter ranges between 0.1 and 0.8 micrometres, and the thickness of the composite fibrous matrix (14) is in the range between 50 and 500 micrometres.

6. The method according to any claim of 1 to 5, **wherein** the aerosol particle (14) sampling (1) by the fibrous matrix (14) comprises:
• preparing (1.1) the fibrous matrix (14) for sampling, preferably, conditioning under temperature 20-22 °C and humidity 40-50%, followed by weighing in a microbalance;
• transferring (1.2) the fibrous matrix (14) to the sampling location of aerosol particles, for example, by placing the fibrous matrix (14) in a sterile Petri-dish;
• inserting (1.3) the fibrous matrix (14) to a designated aerodynamic aerosol particle sampler device, preferably, a cassette, an impactor, or a cyclone;
• drawing (1.4) a defined volume of a sampled aerosol through the fibrous matrix (14), for example, using a vacuum pump at a defined flow rate specific to the sampling device.
• removing (1.5) the fibrous matrix (14) and transferring in a sterile Petri dish under freezing conditions of above -18 °C to prevent evaporation of samples semi-volatile species.

7. The method according to claim 6, **wherein** the volume of air drawn in the drawing (1.4) step is determined by the concentration of aerosol particle in ambient/occupational environment or emission process, to obtain the deposit of aerosol particles (21) in the fibrous matrix (14) within the interval of 20 to 500ug.

8. The method according to claim 7, **wherein** the aerosol particle concentration in the ambient/occupational environment or emission process is estimated by carrying out an initial aerosol sampling or measuring real-time aerosol particle concentration using a direct reading method and its corresponding instruments.

9. The method of any of claims 1 to 5, **wherein** preparation (2) of collected samples of fibrous matrix (14) containing aerosol particles comprises steps of:
• optionally, conditioning (2.1) the fibrous matrix (14) with collected particles (21) under controlled temperature and humidity, followed by weighing;
• preparing (2.2) a circle-shaped discs fibrous matrix (14) with collected particles (21) of a diameter suitable to a cell culture well/vessel and/or cell culture insert, such as 96-, 48-, 24-, 12- well plate format, under sterile conditions, for example, with a sterile mechanical hole puncher under laminar flow hood;
• optionally, storing the fibrous matrix (14) with collected particles (21) until cytotoxicity tests under freezing conditions below -18 °C to prevent the evaporation of volatile particles;
• transferring (2.3) the fibrous matrix (14) with collected particles (21) into the sterile cell culture plate.

10. The method of any of claims 1 to 5, **wherein** cell cultivation (3) on collected samples of fibrous matrix (14) with collected particles (21) for cytotoxicity testing comprises steps of:
• preparing (3.1) a cell suspension from a healthy and viable cell culture, following a standardized sequence, such as
∘ thawing the cells if they are frozen; centrifuging the cells to pellet them; discarding the supernatant, and resuspending the cells in a suitable growth medium; counting the cells using a hemacytometer or automated cell counter, and adjusting the cell density to the desired concentration; transferring the cell suspension to a sterile culture vessel; incubating cells at the appropriate temperature, humidity, and CO₂ level; harvesting for seeding, when the cells reach the desired density or confluency;
∘ selecting appropriate volume/concentration of cell suspension depending on the diameter of the fibrous matrix (14) and deposited amount of aerosol particles (21), as well as selected protocol of cell cultivation;
• seeding (3.2) the cells (33) in a cell culture medium directly onto the fibrous matrix (14) with collected particles (21), optionally, the cell culture medium is supplemented with Fetal Bovine Serum/ Fetal Calf Serum, antibiotics/antimycotics;
• placing (3.3) cell culture vessel with fibrous matrix (14) with collected particles (21) and the cells (33) into cell culture incubator with a specific temperature, humidity and air, for example, 37°C, 21 % O₂, 5 % CO₂;
• allowing (3.4) the cells (33) to attach to fibrous matrix (14) with collected particles (21) within time range from 3 to 24 hours.

11. The method of any of claims 1 to 5, **wherein** cell cytotoxicity quantification (4) on collected samples of fibrous matrix (14) with collected particles (21) for cytotoxicity testing comprises steps any of
• (4.1) adding the cell viability/cytotoxicity reagents, and/or cell dyes into the wells and perform the readings of the signals directly from the culture wells by spectrometer for absorbance, fluorescence, luminescence, or collect/transfer the medium/supernatants from the cultured cells on fibrous matrix (14) with collected particles (21) for viability/cytotoxicity tests;
• visualising (4.2) live/dead cells on fibrous matrix (14) with collected particles (21) by fluorescence/confocal microscope after staining;
• collecting (4.3) the cell culture medium/supernatants and testing by a secreted biomarker;
• lysing (4.4.) cells grown on fibrous matrix (14) with collected particles (21), and extracting RNA/DNA/proteins thereof for a biomarker analysis, such as gene/protein expression, DNA modifications.

12. The method of any of claims 1 to 11, w her e i n target cells for the cytotoxicity testing described include any type of adherent cells derived from tissues of organs, such as but not limited to human bronchial epithelial cells, human lung carcinoma cells, human liver carcinoma cells, human umbilical vein endothelial cells, murine macrophage cells, human neuroblastoma cells, rat kidney epithelial cells.

13. The method of any of claims 1 to 12, used for estimating the toxicity of the ambient air constituents in environmental or occupational pollution research and investigations, such as performed by environmental and health institutions.

14. A cytotoxicity estimation kit comprising a plate of cell cultivation wells, and at least one on fibrous matrix (14) according to any of claims 2 to 4.

15. The on fibrous matrix (14) according to any one of claims 2 to 4, having undergone at least the two first steps (1) and (2) of the method by claims 1 to 8, wherein the on fibrous matrix (14) has been used as the aerosol particle collecting filter and is prepared as a substrate for cell cultivation.
